# EUROPÄISCHE PATENTANMELDUNG

(11) **EP 1 203 820 A1**
(43) Veröffentlichungstag der Anmeldung: **08.05.2002**
(21) Anmeldenummer: 01124665.9
(22) Anmeldetag: 16.10.2001
(51) Int. Cl.: C12P 13/00

(54) **Verfahren zur Herstellung von optisch aktiven Cyanhydrinen und Folgeprodukte**

(30) Priorität: 01.11.2000 DE 10054011; 14.12.2000 DE 10062306
(71) Anmelder: Clariant GmbH, 65929 Frankfurt am Main (DE)
(72) Erfinder: Kirschbaum, Bettina, Dr., 65929 Frankfurt am Main (DE); Wilbert, Götz, Dr., 86368 Gersthofen (DE); Effenberger, Franz, Prof. Dr., 70597 Stuttgart (DE); Brand, Stefan Dr., 69198 Schriesheim (DE); Fromm, Andreas, 60326 Frankfurt (DE); Bühler, Holger Dr, 71640 Ludwigsburg (DE)

(57) **Zusammenfassung**

Die vorliegende Erfindung betrifft ein Verfahren zur Herstellung optisch aktiver Cyanhydrine der Formel (II), 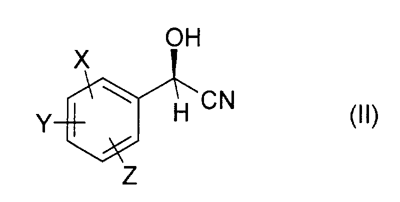 indem man einen Aldehyd der Formel (I) 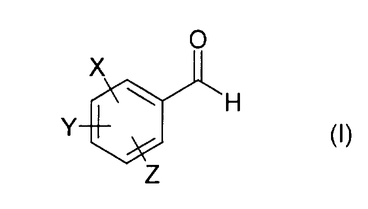 mit HCN in einem mit Wasser nicht mischbaren organischen Lösungsmittel in Anwesenheit von Wasser, in Abwesenheit oder Anwesenheit eines Puffers, in Anwesenheit einer (R)-Hydroxynitril Lyase umsetzt, wobei X, Y und Z in Formel (II) die gleiche Bedeutung wie in Formel (I) haben, unabhängig voneinander gleich oder verschieden sind und für H, F, Cl, Br, I, OH, O(C₁-C₄-Alkyl), OCOCH₃, NHCOCH₃, NO₂ oder C₁-C₄-Alkyl stehen.

## Beschreibung

Die Erfindung betrifft ein Verfahren zur Herstellung von optisch aktiven Cyanhydrinen mittels eines hochaktiven (R)-Hydroxynitril Lyase-Extraktes. Optisch aktive Cyanhydrine und deren Folgeprodukte, wie z. B. optisch aktive α-Hydroxycarbonsäuren, dienen als Bausteine für die Gewinnung biologisch wirksamer Stoffe, die z.B. in der Pharma- oder Agro-Industrie Einsatz finden, wie in F. Effenberger: Hydroxynitrile Lyases in Stereoselective Synthesis (in Stereoselective Biocatalysis edited by R.N. Patel; Marcel Dekker Inc. New York-Basel 2000, Seiten 321 bis 342) beschrieben.
Eine Synthesemöglichkeit für optisch aktive Cyanhydrine bietet der Einsatz von aus Naturprodukten gewonnener Hydroxynitril Lyase, die je nach Naturprodukt in der R-oder S-Form gewonnen werden kann, und Aldehyde in Anwesenheit von Blausäure in entsprechende (R)- oder (S)-Cyanhydrine umwandelt. Das Substratspektrum des Enzyms ist je nach Naturprodukt verschieden. (R)-Hydroxynitril Lyase wird am einfachsten und daher am häufigsten aus Mandelmehl gewonnen.
In der Reihe der aromatischen Aldehyde zählen Benzaldehyd sowie in 3- und 4-Stellung substituierte Benzaldehyde zu den Substraten, die sich mittels aus Mandelmehl als Naturprodukt oder anderen Naturprodukten gewonnener (R)-Hydroxynitril Lyase mit gutem Erfolg zu optisch aktiven Cyanhydrinen umsetzen lassen. Dagegen gehören 4-Hydroxybenzaldehyd und 3,4-Dihydroxybenzaldehyd zu den Einsatzstoffen, die sich mittels aus Mandelmehl oder anderen Naturprodukten gewonnener (R)-Hydroxynitril Lyase nur schwierig in die entsprechenden optisch aktiven Cyanhydrine umsetzen lassen. In 2-Stellung substituierte Benzaldehyde sind als Substrate für die Umsetzung mittels (R)-Hydroxynitril Lyasen wenig geeignet.

Ein Nachteil der bekannten Verfahren besteht darin, dass bei der Umsetzung der Aldehyde mittels (R)-Hydroxynitril Lyase mit HCN bzw. KCN zu den entsprechenden optisch aktiven Cyanhydrinen die Aldehyde üblicherweise in Form verdünnter Lösungen geringer Konzentration eingesetzt werden. Dementsprechend niedrig fällt die Raumzeitausbeute bezogen auf die eingesetzten Aldehyde aus. Im Hinblick auf die vorstehend geschilderten Einschränkungen bezüglich der einzusetzenden Aldehyde und die Nachteile niedriger Raumzeitausbeuten besteht der Bedarf an einem Verfahren, das diese Einschränkungen und Nachteile vermeidet und sich darüber hinaus auf einfache Art, ohne großen Aufwand zu erfordern, auch technisch realisieren lässt.

Diese Aufgabe wird überraschenderweise gelöst durch ein Verfahren zur Herstellung optisch aktiver Cyanhydrine der Formel (II)

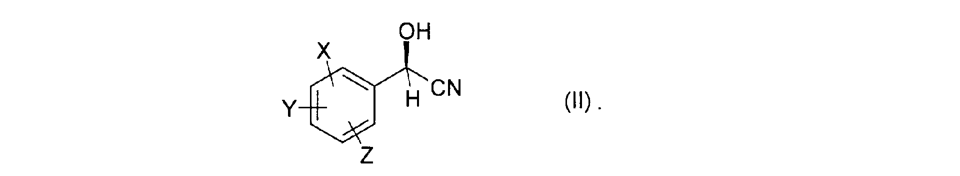

Es ist dadurch gekennzeichnet, dass man einen Aldehyd der Formel (I) 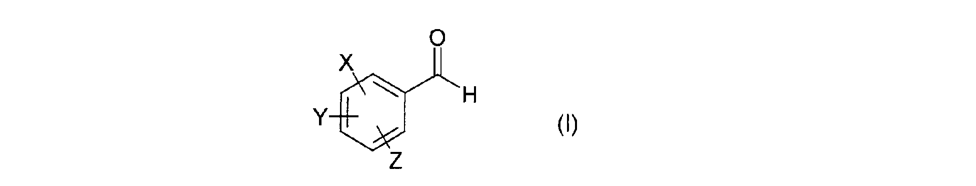 mit HCN in einem mit Wasser nicht mischbaren organischen Lösungsmittel in Anwesenheit von Wasser, in Abwesenheit oder Anwesenheit eines Puffers, in Anwesenheit einer (R)-Hydroxynitril Lyase umsetzt, wobei X, Y und Z in Formel (II) die gleiche Bedeutung wie in Formel (I) haben, unabhängig voneinander gleich oder verschieden sind und für H, F, Cl, Br, I, OH, O(C₁-C₄-Alkyl), OCOCH₃, NHCOCH₃, NO₂ oder C₁-C₄-Alkyl stehen.

Während der gesamten Umsetzung mit HCN hält man üblicherweise einen pH-Wert von 0 bis 8, insbesondere 2 bis 7, bevorzugt 3 bis 6 ein. In einer Reihe von Fällen hat es sich als vorteilhaft erwiesen einen pH-Wert von 3,3 bis 5,5, insbesondere 4,0 bis 5,5, bevorzugt 4,5 bis 5,3 einzuhalten.

Man führt die Umsetzung üblicherweise bei 0 bis 60°C durch und arbeitet unter intensiver Durchmischung. Das Cyanhydrin der Formel (II) lässt sich gegebenenfalls durch Hydrolyse in die entsprechende Carbonsäure überführen.

Die nachstehenden Ausführungen, die auch im Zusammenhang mit einer Arbeitsweise unter Einsatz eines wässrigen R-Hydroxynitril Lyase-Extrakts stehen, treffen auch auf die vorstehend genannte Arbeitsweise in Anwesenheit einer R-Hydroxynitril Lyase zu. Die Arbeitsweise in Anwesenheit des nachstehend erwähnten wässrigen R-Hydroxynitril Lyase-Extraktes stellt eine spezielle Variante des erfindungsgemäßen Verfahrens in Anwesenheit einer R-Hydroxynitril Lyase dar. Die R-Hydroxynitril Lyase kann in reiner Form oder in Form eines Extraktes eingesetzt werden. Die R-Hydroxynitril Lyase ist beispielsweise in reiner bzw. gereinigter Form kommerziell erhältlich.

Man führt die Umsetzung üblicherweise in Anwesenheit von 20 bis 1000 Units R-Hydroxynitril Lyase/mmol Aldehyd, insbesondere 50 bis 500 Units R-Hydroxynitril Lyase/mmol Aldehyd, bevorzugt 80 bis 400 Units R-Hydroxynitril Lyase/mmol Aldehyd durch. Die für die Umsetzung des Aldehyds erforderliche Menge Units R-Hydroxynitril Lyase hängt auch von der Art des Aldehyds ab. Leicht umzusetzende Aldehyde lassen sich mit relativ niedrigen Mengen Units R-Hydroxynitril Lyase/mmol Aldehyd umsetzen, während weniger reaktive Aldehyde höhere Mengen Units R-Hydroxynitril Lyase/mmol Aldehyd erfordern. Die Units (Abkürzung U) sind ein Maß für die Aktivität der R-Hydroxynitril Lyase.

Wie bereits erwähnt führt man die Umsetzung in Abwesenheit oder Anwesenheit eines Puffers durch. Die Umsetzung in Abwesenheit eines Puffers stellt eine besonders einfache Variante des erfindungsgemäßen Verfahrens dar.

In einer Reihe von Fällen kann es von Vorteil sein, die erfindungsgemäße Umsetzung des Aldehyds mit HCN in Anwesenheit eines Puffers durchzuführen. Besonders geeignet sind Puffer bzw. Puffergemische, die ihre Pufferwirkung in dem angegebenen pH-Bereich entfalten und den pH-Wert während der Umsetzung in diesem Bereich halten. Reicht die puffernde Wirkung des Puffers nicht aus, den pH-Wert während der Umsetzung in dem vorgegebenen Bereich zu halten, muss erforderlichenfalls durch Säurezugabe oder Basenzugabe der pH-Wert eingestellt werden.
Geeignete Puffer sind beispielsweise Glutaminsäure-Glutamat-, Phosphorsäure-Phosphat-, Essigsäure-Acetat- und Citronensäure-Citrat-Puffer, insbesondere Essigsäure-Acetat- und Citronensäure-Citrat-Puffer.
Es hat sich als nützlich erwiesen, die Umsetzung in Anwesenheit von 20 bis 500 mmol Puffer/Liter, insbesondere 40 bis 300 mmol Puffer/Liter, bevorzugt 80 bis 160 mmol Puffer/Liter durchzuführen. Üblicherweise löst man den Puffer in Wasser und setzt ihn in Form einer 20 bis 500, insbesondere 40 bis 300, bevorzugt 80 bis 160 mmol Puffer je Liter enthaltenden wässrigen Lösung ein.

Die Erfindung betrifft nach einer besonderen Ausführungsform ein Verfahren zur Herstellung optisch aktiver Cyanhydrine der Formel (II)

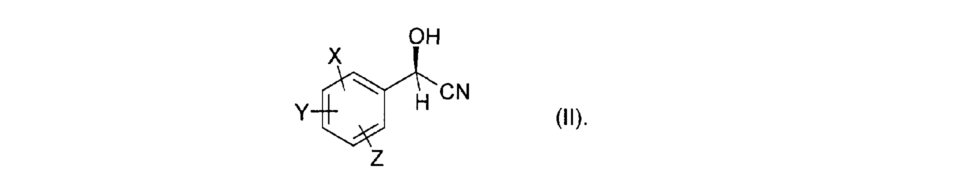

Es ist dadurch gekennzeichnet, dass man einen Aldehyd der Formel (I) 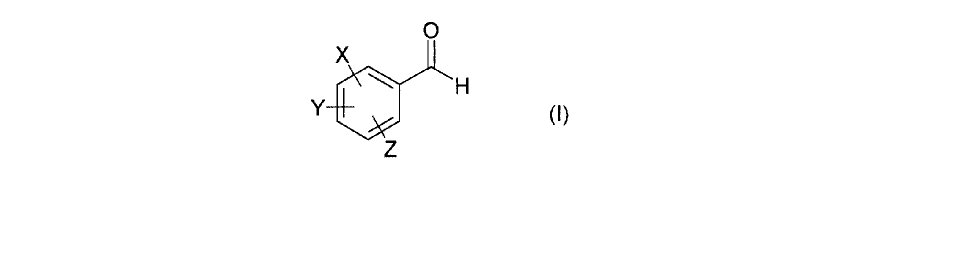 mit HCN in einem mit Wasser nicht mischbaren organischen Lösungsmittel in Anwesenheit eines wässrigen, durch Extraktion eines (R)-Hydroxynitril Lyase enthaltenden Naturproduktes bei pH 3,3 bis 5,5 mit Wasser, in Abwesenheit oder Anwesenheit eines Puffers, hergestellten (R)-Hydroxynitril Lyase-Extraktes unter intensiver Durchmischung bei 0 bis 60°C umsetzt, die organische Phase von der wässrigen Phase trennt und gegebenenfalls das Cyanhydrin der Formel (II) durch Hydrolyse in die entsprechende Carbonsäure überführt, wobei X, Y und Z in Formel (II) die gleiche Bedeutung wie in Formel (I) haben, unabhängig voneinander gleich oder verschieden sind und für H, F, Cl, Br, I, OH, O(C₁-C₄-Alkyl), OCOCH₃, NHCOCH₃, NO₂ oder C₁-C₄-Alkyl stehen.
Das erfindungsgemäße Verfahren ermöglicht es, überraschenderweise auch schwer umzusetzende Aldehyde, wie 4-Hydroxybenzaldehyd und 3,4-Dihydroxybenzaldehyd, mit hohen Umsätzen und guten ee-Werten in die optisch aktiven Cyanhydrine zu überführen. Darüber hinaus lassen sich auch in 2-Stellung substituierte Benzaldehyde, beispielsweise 2-Chlorbenzaldehyd, mit gutem Erfolg mittels des erfindungsgemäßen Verfahrens zu den entsprechenden optisch aktiven Cyanhydrinen umsetzen. Im Hinblick darauf, dass bei einer Umsetzung von in 2-Stellung substituierten Benzaldehyden zu optisch aktiven Cyanhydrinen wegen der sterischen Verhältnisse wohl erhebliche Schwierigkeiten zu erwarten waren, ist es als sehr überraschend anzusehen, dass auch in 2-Stellung substituierte Benzaldehyde sich in die optisch aktiven Cyanhydrine umwandeln lassen.
Ein weiterer Vorteil des erfindungsgemäßen Verfahrens besteht darin, dass es möglich ist, die Aldehyde nicht nur wie bislang üblich in vergleichsweise niedrig konzentrierten Mengen, beispielsweise 0,1 mol Aldehyd/Liter, einzusetzen, sondern auch die Umsetzung mit erheblich höheren Aldehydkonzentrationen, beispielsweise 1,0 mol Aldehyd/Liter und höher, durchzuführen. Dementsprechend fällt auch die Raumzeitausbeute höher aus und erreicht für Enzymreaktionen unerwartet hohe Werte.

Mit gutem Erfolg kann man in das erfindungsgemäße Verfahren einen Aldehyd der Formel (la) 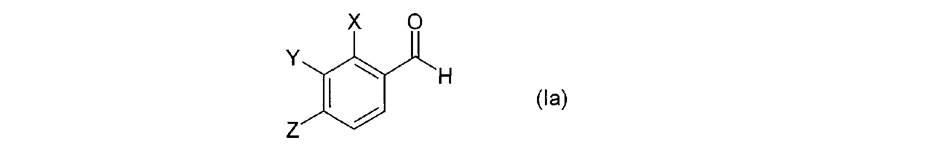 einsetzen, worin X, Y und Z die gleiche Bedeutung wie in den vorstehend genannten Formeln (I) und (II) besitzen.
Insbesondere kann man einen Aldehyd der Formel (la), worin X für F, Cl, Br, I, OH, O(C₁-C₄-Alkyl), OCOCH₃, NHCOCH₃, NO₂ oder C₁-C₄-Alkyl, insbesondere für F, Cl, Br, OH, O(C₁-C₄-Alkyl) oder C₁-C₄-Alkyl, bevorzugt für F, Cl, OH, O(C₁-C₄-Alkyl) oder C₁-C₄-Alkyl, und Y und Z jeweils für H stehen oder X und Y jeweils für H und Z für OH steht oder X für H und Y und Z jeweils für OH steht, einsetzen.
Von Interesse ist ein Aldehyd der Formal (la), worin X für F, Cl, OH, O(C₁-C₂-Alkyl) oder C₁-C₂-Alkyl und Y und Z jeweils für H stehen oder X und Y jeweils für H und Z für OH stehen oder X für H und Y und Z jeweils für OH stehen.
Man führt die Umsetzung mit HCN in einem mit Wasser nicht mischbaren organischen Lösungsmittel durch. Hierfür eignen sich prinzipiell alle organischen Lösungsmittel oder Lösungsmittelgemische, die sich unter den Bedingungen der Reaktion inert verhalten. Besonders geeignet sind Lösungsmittel oder Lösungsmittelgemische, die geringe Mengen Wasser, beispielsweise 0,3 bis 5,0, insbesondere 0,5 bis 3,0, bevorzugt 0,6 bis 2,5 Gew.-% Wasser, bezogen auf organisches Lösungsmittel, lösen.
Man kann als mit Wasser nicht mischbares organisches Lösungsmittel einen aliphatischen Ether, insbesondere einen Dialkylether mit 1 bis 5, bevorzugt 2 bis 4 Kohlenstoffatomen je Alkylrest, einen Ester einer Carbonsäure, insbesondere einer aliphatischen Carbonsäure, mit 1 bis 6, insbesondere 1 bis 4 Kohlenstoffatomen und eines aliphatischen Alkohols mit 1 bis 4, insbesondere 1 bis 2 Kohlenstoffatomen, ein aliphatisches Keton mit insgesamt 4 bis 8, insbesondere 4 bis 6 Kohlenstoffatomen oder ein Gemisch derselben oder eine verdünnte Lösung derselben mit einem unpolaren organischen Lösungsmittel, beispielsweise mit einem aliphatischen Kohlenwasserstoff mit 4 bis 8, insbesondere 4 bis 6 Kohlenstoffatomen, einem aromatischen Kohlenwasserstoff mit 7 bis 10, insbesondere 7 bis 9 Kohlenstoffatomen, wie Toluol, ortho-, meta- und/oder para-Xylol, einem chlorierten aliphatischen oder aromatischen Kohlenwasserstoff wie Methylenchlorid, Dichlorethan, Trichlorethan, Chloroform, Chlorbenzol, Dichlorbenzol und Trichlorbenzol einsetzen.

Man kann mit gutem Erfolg als mit Wasser nicht mischbares Lösungsmittel Diethylether, Di-n-propylether, Di-i-propylether, Methyl-tert.-butylether, Di-n-butylether, Di-i-butylether oder ein Gemisch derselben, insbesondere Diethylether, Di-n-propylether, Di-i-propylether, Methyl-tert.-butylether oder ein Gemisch derselben, bevorzugt Di-i-propylether oder Methyl-tert.-butylether einsetzen.
Man setzt je mol Aldehyd der Formel (I) bzw. (la) 0,8 bis 10,0, insbesondere 1,0 bis 5,0, bevorzugt 1,2 bis 2,0, besonders bevorzugt 1,3 bis 1,7 mol HCN ein. Es ist jedoch auch möglich die Umsetzung bei 0,5 bis 20 mol HCN / mol Aldehyd durchzuführen.

Wie eingangs bereits erwähnt, führt man die Umsetzung in Anwesenheit einer R-Hydroxynitril Lyase bzw. eines wässrigen, durch Extraktion eines (R)-Hydroxynitril Lyase enthaltenden Naturprodukts bei pH 3,3 bis 5,5 mit Wasser, in Abwesenheit oder Anwesenheit eines Puffers, hergestellten (R)-Hydroxynitril Lyase-Extraktes durch.
Der Vollständigkeit halber sei an dieser Stelle auf die Herstellung des (R)-Hydroxynitril Lyase-Extraktes, etwas näher eingegangen.
Der (R)-Hydroxynitril Lyase-Extrakt wird durch ein Verfahren zur Herstellung eines (R)-Hydroxynitril Lyase-Extraktes durch Extraktion eines (R)-Hydroxynitril Lyase enthaltenden Naturproduktes mit Wasser, in Abwesenheit oder Anwesenheit eines Puffers, bei einem pH-Wert von 3,3 bis 5,5 zugänglich gemacht.
Das Verfahren ist eine einfache und technisch ohne besonderen Aufwand realisierbare Herstellungsmethode für einen (R)-Hydroxynitril Lyase-Extrakt.
Man setzt als (R)-Hydroxynitril Lyase enthaltendes Naturprodukt mit gutem Erfolg zerkleinerte Fruchtkerne, insbesondere zerkleinerte, entfettete Fruchtkerne ein. Unter Fruchtkernen versteht man Obstkerne und Obststeine.
Nach einer besonders geeigneten Variante setzt man als (R)-Hydroxynitril Lyase enthaltendes Naturprodukt zerkleinerte, entfettete Apfel- oder Mandelkerne ein. Es lassen sich jedoch auch andere (R)-Hydroxynitril Lyase enthaltende Obstkerne oder Obststeine, die vorzugsweise zerkleinert und entfettet sind, in dieses Verfahren einsetzen.
Das Verfahren verwendet nach einer bevorzugten Variante entfettetes Mandelmehl, so wie es bei der Herstellung von Mandelöl als Abfallprodukt anfällt.
Die Enzymextraktion wird bei einer Temperatur von 0 bis 60°C, vorzugsweise 10 bis 50°C, insbesondere 20 bis 40°C durchgeführt.
Wie vorstehend bereits erwähnt, wird die Extraktion des (R)-Hydroxynitril Lyase enthaltenden Naturproduktes mit Wasser, in Abwesenheit oder Anwesenheit eines Puffers, durchgeführt. Arbeitet man ohne Puffer, so muss man Sorge dafür tragen, dass der vorgegebene pH-Wert von 3,3 bis 5,5 während der gesamten Extraktion eingehalten wird. Dies erreicht man, da während der Extraktion der pH-Wert auf Werte > 5,5 ansteigt, durch eine kontrollierte Zugabe von Säure, beispielsweise einer Mineralsäure, während der Extraktion. Das für die Extraktion verwendete Wasser wird zuvor auch durch Säurezugabe auf einen entsprechenden pH-Wert eingestellt. Eine derartige Arbeitsweise ist Beispiel 5 zu entnehmen. pH-Werte oberhalb 5,5 führen zu Extrakten mit verringerter Wirkung in der enantioselektiven Cyanhydrinreaktion, siehe Beispiel 4a (Vergleichsbeispiel).
Man setzt üblicherweise das (R)-Hydroxynitril Lyase enthaltende Naturprodukt und Wasser bzw. wässrige Pufferlösung im Gewichtsverhältnis 1 : (1 bis 50), insbesondere 1 : (2 bis 20), bevorzugt 1 : (2,5 bis 10) ein.
In einer Reihe von Fällen kann es von Vorteil sein, die Extraktion des (R)-Hydroxynitril Lyase enthaltenden Naturproduktes in Anwesenheit eines Puffers durchzuführen. Besonders geeignet sind Puffer bzw. Puffergemische, die ihre Pufferwirkung in dem angegebenen pH-Bereich von 3,3 bis 5,5 entfalten und den pH-Wert während der Extraktion in diesem Bereich halten. Reicht die puffernde Wirkung des Puffers nicht aus, den pH-Wert während der Extraktion in dem vorgegebenen Bereich zu halten, muss ebenfalls durch Säurezugabe der pH-Wert eingestellt werden.
Geeignete Puffer sind beispielsweise Glutaminsäure-Glutamat-, Phosphorsäure-Phosphat-, Essigsäure-Acetat- und Citronensäure-Citrat-Puffer, insbesondere Essigsäure-Acetat- und Citronensäure-Citrat-Puffer.
Es hat sich als nützlich erwiesen, die Extraktion in Anwesenheit von 20 bis 500 mmol Puffer/Liter, insbesondere 40 bis 300 mmol Puffer/Liter, bevorzugt 80 bis 160 mmol Puffer/Liter durchzuführen. Üblicherweise löst man den Puffer in Wasser und setzt ihn in Form einer 20 bis 500, insbesondere 40 bis 300, bevorzugt 80 bis 160 mmol Puffer je Liter enthaltenden wässrigen Lösung ein.
Die Extraktion gestaltet sich besonders einfach, wenn der pH-Wert des Puffers und die Menge des Puffers so gewählt werden, dass sichergestellt ist, dass der vorgegebene pH-Bereich während der gesamten Extraktion eingehalten wird.
Das Naturprodukt kann auch in nicht entfetteter Form eingesetzt werden. Hierbei ist dessen Anteil im Verhältnis zur Pufferlösung entsprechend dem Fettgehalt zu erhöhen.
Nach einer angemessenen Einwirkdauer des Wassers bzw. der Pufferlösung auf das Naturprodukt von beispielsweise 0,5 bis 24, insbesondere 2 bis 20, bevorzugt 3 bis 18 Stunden, wird der Enzymextrakt üblicherweise durch Filtration mit einem geeigneten Filterapparat vom Naturprodukt getrennt.
Man kann den wässrigen Enzymextrakt zusammen mit dem extrahierten Naturprodukt für die enantioselektive HCN-Addition verwenden. Es ist jedoch günstig, das extrahierte Naturprodukt abzutrennen und den wässrigen, vom Naturprodukt befreiten Enzymextrakt zu benutzen. Auf diese Weise vermeidet man durch HCN verunreinigtes Naturprodukt als Abfall.

Zur Durchführung des erfindungsgemäßen Verfahrens wird die wässrige Phase, die die R-Hydroxynitril Lyase respektive den (R)-Hydroxynitril Lyase-Extrakt enthält, mit der organischen Phase, in der der Aldehyd der Formel (I) respektive (la) gelöst ist, intensiv vermischt. Das HCN verteilt sich entsprechend der Löslichkeit sowohl auf die wässrige als auch auf die organische Phase. Durch das Vermischen wird gewährleistet, dass der Aldehyd mit der (R)-Hydroxynitril Lyase in Kontakt kommt und wunschgemäß mit dem HCN zu dem entsprechenden optisch aktiven Aldehyd reagiert.
Man führt die Umsetzung, wie eingangs erwähnt, bei 0 bis 60°C, insbesondere bei 10 bis 50°C, bevorzugt bei 20 bis 40°C durch. In vielen Fällen hat es sich bewährt, die Reaktion bei Raumtemperatur ablaufen zu lassen.
Man setzt den Aldehyd der Formel (I) respektive (la) in einer Konzentration von 0,1 bis 3,0, insbesondere 0,5 bis 3,0, bevorzugt 1,0 bis 3,0 mol Aldehyd/Liter ein. In einer Vielzahl von Fällen führt man die Umsetzung mit HCN mit einer Aldehydkonzentration von 1,5 bis 2,5 mol/Liter durch.
Während der gesamten Umsetzung des Aldehyds mit HCN in Gegenwart der R-Hydroxynitril Lyase respektive des (R)-Hydroxynitril Lyase-Extraktes hält man - erforderlichenfalls durch Zugabe von Säure oder Base - einen pH-Wert von 3,3 bis 5,5, insbesondere 4,0 bis 5,5, bevorzugt 4,5 bis 5,3 ein.

Das Gewichtsverhältnis von organischer Phase zu wässriger Phase (Enzymextrakt) beträgt üblicherweise 20 : 1 bis 1 : 20, insbesondere 10 : 1 bis 1 : 10, bevorzugt 5 : 1 bis 1 : 5, besonders bevorzugt 2 : 1 bis 1 : 2.
Nach Beendigung der Umsetzung lässt man die Phasen sich trennen. Die organische Phase enthält das optisch aktive Cyanhydrin, und in der wässrigen Phase befindet sich die (R)-Hydroxynitril Lyase, die in die Reaktion zurückgeführt werden kann.
Anschließend kann man - falls gewünscht - das optisch aktive Cyanhydrin aus der organischen Phase abtrennen und gegebenenfalls noch reinigen. Man kann aber auch das optisch aktive Cyanhydrin, gegebenenfalls in Form der organischen Phase, beispielsweise durch saure Hydrolyse in die entsprechende optisch aktive α-Hydroxycarbonsäure (α-Hydroxyphenylessigsäure = Mandelsäure) überführen. Für die saure Hydrolyse verwendet man üblicherweise starke Mineralsäuren, wie konz. HCI oder wässrige Schwefelsäure. Bei der Hydrolyse ist ebenfalls für eine gute Durchmischung der wässrigen Phase, in der die Säure enthalten ist, und der organischen Phase, in der sich das optisch aktive Cyanhydrin befindet, zu sorgen.

Die nachfolgenden Beispiele beschreiben die Erfindung näher, ohne sie zu beschränken.

Die Aktivitäten der hergestellten Enzymextrakte wurden nach einer Methode von M. Bauer, H. Griengl und W. Steiner Biotechnol. Bioeng. 1999, 62, 23 bestimmt. Die ee-Werte der erhaltenen Cyanhydrine wurden nach Derivatisierung mit Acetanhydrid / Pyridin gaschromatographisch über eine β-Cyclodextrin-Säule bestimmt. (Definition 1 Unit siehe auch K. Drauz, H. Waldmann Enzyme Catalysis in Organic Synthesis, Vol. I, Verlag Chemie, Weinheim, 1995, S. 22.)

### Experimenteller Teil:

Unter Enzym wird nachfolgend stets (R)-Hydroxynitril Lyase verstanden.

### Beispiel 1:

### Herstellung von Enzymextrakt mit 80 mmol Citrat-Puffer/Liter, pH 4,8

8,4 g Citronensäure-Monohydrat werden mit demineralisiertem Wasser auf 500 ml aufgefüllt. Mit wenigen Tropfen 50 % NaOH-Lösung wird der pH auf 4,8 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,2 und einer Aktivität von etwa 200 U/ml.

### Herstellung von (R)-2-Chlorbenzaldehydcyanhydrin

56,2 g 2-Chlorbenzaldehyd (0,4 mol) werden in 200 ml Diisopropylether gelöst und mit 200 ml des vorstehend hergestellten Enzymextraktes (40000 U) und 16,2 g HCN (0,6 mol) versetzt. Die Reaktionsmischung wird 45 min bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 99 %), mit einem ee von 83 %.

### Beispiel 2:

### Herstellung von Enzymextrakt mit 80 mmol Citrat-Puffer/Liter, pH 3,3

8,4 g Citronensäure-Monohydrat werden mit demineralisiertem Wasser auf 500 ml aufgefüllt. Mit wenigen Tropfen 50 % NaOH-Lösung wird der pH auf 3,3 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 4,4 und einer Aktivität von etwa 75 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 270 ml des vorstehend hergestellten Enzymextraktes (20000 U) und 8,1 g HCN (0,3 mol) versetzt. Die Reaktionsmischung wird 90 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 95 %), mit einem ee von 82 %.

### Beispiel 3:

### Herstellung von Enzymextrakt mit 160 mmol Citrat-Puffer/Liter, pH 4,8

16,8 g Citronensäure-Monohydrat werden mit demineralisiertem Wasser auf 500 ml aufgefüllt. Mit wenigen Tropfen 50 % NaOH-Lösung wird der pH auf 4,8 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,0 und einer Aktivität von etwa 200 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 100 ml des vorstehend hergestellten Enzymextraktes (20000 U) und 8,1 g HCN (0,3 mol) versetzt. Die Reaktionsmischung wird 45 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 98 %), mit einem ee von 83 %.

### Beispiel 4:

### Herstellung von Enzymextrakt mit 20 mmol Citrat-Puffer/Liter, pH 3,3

2,1 g Citronensäure-Monohydrat werden mit demineralisiertem Wasser auf 500 ml aufgefüllt. Mit wenigen Tropfen 50 % NaOH-Lösung wird der pH auf 3,3 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,0 und einer Aktivität von etwa 70 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 285 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 98 %), mit einem ee von 83 %.

### Beispiel 4a (Vergleichsbeispiel):

Herstellung von Enzymextrakt mit 20 mmol Citrat-Puffer/Liter, pH 5,5 (in Anlehnung an das in Synth. Commun. 1991, 21, Seite 1388 beschriebene Verfahren, jedoch mittels Extraktion und Abtrennung von Mandelmehl)
2,1 g Citronensäure-Monohydrat werden mit demineralisiertem Wasser auf 500 ml aufgefüllt. Mit wenigen Tropfen 50 % NaOH-Lösung wird der pH auf 5,5 eingestellt. 100 g entfettetes Mandelmehl werden mit 500 ml dieses Citrat-Puffers versetzt und 16 Stunden bei Raumtemperatur gerührt. Der pH-Wert steigt während der Extraktion - wie nachfolgend bei dem erhaltenen Enzymextrakt ausgewiesen - deutlich an. Es wird über eine Glasfritte filtriert. Man erhält etwa 400 ml wässrigen Enzymextrakt, mit einem pH von 6,0 und einer Aktivität von etwa 70 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 285 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die organische Phase enthält (R)-2-Chlorbenzaldehydcyanhydrin (Umsatz laut GC 99 %), mit einem ee von 55 %.
Die Wirkung des Enzymextraktes ist, wie ein einfacher Vergleich der ee-Werte (ee = enantiomeric excess) bei der Herstellung von (R)-2-Chlorbenzaldehydcyanhydrin in Beispiel 4 deutlich macht, erheblich geringer als bei Verwendung eines erfindungsgemäß hergestellten Enzymextrakts.

### Beispiel 5:

### Herstellung von Enzymextrakt mit wässriger Lösung, pH 4,5 - 5,2 (ohne Puffer)

500 ml demineralisiertes Wasser werden mit wenigen ml konz. HCI auf pH 4,5 eingestellt. 100 g entfettetes Mandelmehl werden mit dieser Lösung versetzt und 16 Stunden bei Raumtemperatur gerührt, wobei der pH-Wert mittels kontinuierlicher Zugabe von konz. HCI in einem pH-Bereich von 4,5 - 5,2 gehalten wird. Es wird über eine Glasfritte filtriert. Man erhält etwa 400 ml wässrigen Enzymextrakt, mit einem pH von 4,8 und einer Aktivität von etwa 200 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 100 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 99 %), mit einem ee von 83 %.

### Beispiel 6:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-Benzaldehydcyanhydrin

74,3 g Benzaldehyd (0,7 mol) werden in 250 ml Diisopropylether gelöst und mit 100 ml des vorstehend genannten Enzymextraktes (20000 U) und 27 g HCN (1,0 mol) versetzt. Die Reaktionsmischung wird 180 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-Benzaldehydcyanhydrin (Umsatz laut GC quantitativ), mit einem ee von 98 %.

### Beispiel 7:

### Herstellung von Enzymextrakt mit 80 mmol Citrat-Puffer / Liter, pH 4,8

Die Herstellung des Enzymextraktes erfolgt wie in Beispiel 1 beschrieben.

### Herstellung von (R)-3-Hydroxybenzaldehydcyanhydrin

12,2 g 3-Hydroxybenzaldehyd (0,1 mol) werden in 100 ml Diisopropylether gelöst und mit 100 ml des vorstehend genannten Enzymextraktes (20000 U) und 4 g HCN (0,15 mol) versetzt. Die Reaktionsmischung wird 105 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3-Hydroxybenzaldehydcyanhydrin (Umsatz laut GC 96 %), mit einem ee von 97 %.

### Beispiel 8:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-3-Hydroxybenzaldehydcyanhydrin

48,8 g 3-Hydroxybenzaldehyd (0,4 mol) werden in 200 ml Diisopropylether gelöst und mit 100 ml des vorstehend genannten Enzymextraktes (20000 U) und 16 g HCN (0,6 mol) versetzt. Die Reaktionsmischung wird 225 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3-Hydroxybenzaldehydcyanhydrin (Umsatz laut GC 94 %), mit einem ee von 92 %.

### Beispiel 9:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-4-Hydroxybenzaldehydcyanhydrin

12,2 g 4-Hydroxybenzaldehyd (0,1 mol) werden in 100 ml Diisopropylether gelöst und mit 100 ml des vorstehend genannten Enzymextraktes (20000 U) und 4 g HCN (0,15 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-4-Hydroxybenzaldehydcyanhydrin (Umsatz laut GC 70 %), mit einem ee von 92 %.

### Beispiel 9a (Vergleichsbeispiel):

### Herstellung von Enzymextrakt mit 80 mmol Citrat-Puffer/Liter, pH 5,3

8,4 g Citronensäure-Monohydrat werden mit demineralisiertem Wasser auf 500 ml aufgefüllt. Mit wenigen Tropfen 50 % NaOH-Lösung wird der pH auf 5,3 eingestellt. 100 g entfettetes Mandelmehl werden mit 500 ml Citrat-Puffer versetzt und 16 Stunden bei Raumtemperatur gerührt. Es wird über eine Glasfritte filtriert. Man erhält etwa 400 ml wässrigen Enzymextrakt, mit einem pH von 5,7 und einer Aktivität von etwa 200 U/ml.

### Herstellung von (R)-4-Hydroxybenzaldehydcyanhydrin

12,2 g 4-Hydroxybenzaldehyd (0,1 mol) werden in 100 ml Diisopropylether gelöst und mit 100 ml des vorstehend hergestellten Enzymextraktes (20000 U) und 4 g HCN (0,15 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die organische Phase enthält (R)-4-Hydroxybenzaldehydcyanhydrin (Umsatz laut GC 63 %), mit einem ee von 70 %.

### Beispiel 10:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-3,4-Dihydroxybenzaldehydcyanhydrin

6,9 g 3,4-Dihydroxybenzaldehyd (0,05 mol) werden in 100 ml Diisopropylether gelöst und mit 100 ml des vorstehend genannten Enzymextraktes (20000 U) und 3 g HCN (0,1 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3,4-Dihydroxybenzaldehydcyanhydrin (Umsatz laut GC 65 %), mit einem ee von 76 %.

### Beispiel 11:

### Herstellung von Enzymextrakt mit 80 mM Citrat-Puffer:

8,4 g Citronensäure-Monohydrat werden mit demineralisiertem Wasser auf 500 ml aufgefüllt. Mit wenigen Tropfen 50 % NaOH-Lösung wird der pH auf 4,8 eingestellt. 100 g entfettetes Mandelmehl werden mit 500 ml Citrat-Puffer versetzt und 16 Stunden bei Raumtemperatur gerührt. Es wird über eine Glasfritte filtriert. Man erhält etwa 400 ml wässrigen Enzymextrakt, mit einem pH von 5,2 und einer Aktivität von etwa 200 U/ml.

### Herstellung von (R)-2-Chlorbenzaldehydcyanhydrin

56,2 g 2-Chlorbenzaldehyd (0,4 mol) werden in 200 ml Diisopropylether gelöst und mit 200 ml des vorstehend beschriebenen Enzymextraktes (40000 U) und 16,2 g HCN (0,6 mol) versetzt. Die Reaktionsmischung wird 45 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 99 %), mit einem ee von 83 %.

### Beispiel 12:

### Herstellung von (R)-2-Chlormandelsäure

Die Diisopropylether-Phase aus Beispiel 11 wird mit 135 g konz. HCI versetzt und unter guter Rührung 6 Stunden auf 60°C erwärmt. Während der Hydrolyse fällt Ammoniumchlorid aus, das nach dem Abkühlen mit wenig Wasser in Lösung gebracht wird. Die Phasen werden getrennt, und die wässrige Phase wird weitere zwei Male mit je 50 ml Diisopropylether extrahiert. Die vereinigten organischen Phasen werden eingeengt, der Rückstand wird in 200 ml Toluol aufgenommen und kurz zum Sieden erhitzt. Beim Abkühlen kristallisiert (R)-2-Chlormandelsäure aus (58,2 g, 78 % d.Th., ee 92 %).
Das Produkt kann durch eine weitere Kristallisation aus Toluol auf eine optische Reinheit von > 99 % gebracht werden.

### Beispiel 13:

### Herstellung von (R)-2-Fluormandelsäure

Entsprechend Beispiel 11 wird 2-Fluorbenzaldehyd (12,4 g, 0,10 mol) in Diisopropylether (50 ml) mit Enzymextrakt aus Beispiel 11 (50 ml) und HCN (4,0 g, 0,15 mol) behandelt. Der Umsatz beträgt nach 60 min > 99 %.
Entsprechend Beispiel 12 wird die Diisopropylether-Phase mit konz. HCI (35 ml) behandelt. Nach Aufarbeitung wird (R)-2-Fluormandelsäure (13,4 g, 81 % d.Th., ee 93 %) erhalten.

### Beispiel 14:

### Herstellung von (R)-2-Brommandelsäure

Entsprechend Beispiel 11 wird 2-Brombenzaldehyd (18,5 g, 0,10 mol) in Diisopropylether (50 ml) mit Enzymextrakt aus Beispiel 11 (50 ml) und HCN (4,0 g, 0,15 mol) behandelt. Der Umsatz beträgt nach 60 min > 95 %.
Entsprechend Beispiel 12 wird die Diisopropylether-Phase mit konz. HCI (35 ml) behandelt. Nach Aufarbeitung wird (R)-2-Brommandelsäure (15,1 g, 63 % d.Th., ee 91 %) erhalten.

## Patentansprüche

1. Verfahren zur Herstellung optisch aktiver Cyanhydrine der Formel (II), 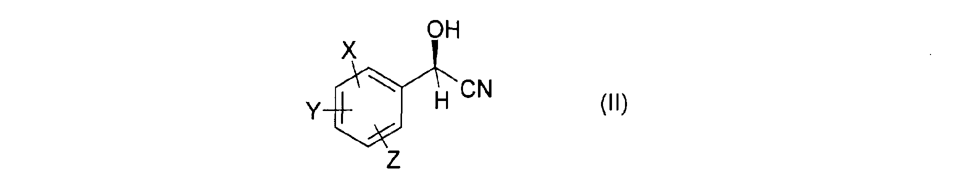 **dadurch gekennzeichnet, dass** man einen Aldehyd der Formel (I) 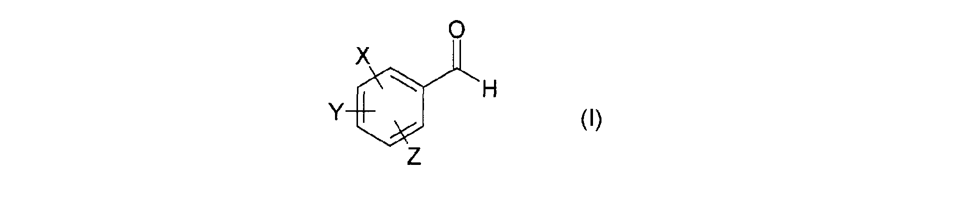 mit HCN in einem mit Wasser nicht mischbaren organischen Lösungsmittel in Anwesenheit von Wasser, in Abwesenheit oder Anwesenheit eines Puffers, in Anwesenheit einer (R)-Hydroxynitril Lyase umsetzt, wobei X, Y und Z in Formel (II) die gleiche Bedeutung wie in Formel (I) haben, unabhängig voneinander gleich oder verschieden sind und für H, F, Cl, Br, I, OH, O(C₁-C₄-Alkyl), OCOCH₃, NHCOCH₃, NO₂ oder C₁-C₄-Alkyl stehen.

2. Verfahren nach Anspruch 1, **dadurch gekennzeichnet, dass** man während der gesamten Umsetzung mit HCN einen pH-Wert von 0 bis 8 einhält.

3. Verfahren nach Anspruch 1 oder 2, **dadurch gekennzeichnet, dass** man während der gesamten Umsetzung mit HCN einen pH-Wert von 2 bis 7 einhält.

4. Verfahren nach einem oder mehreren der Ansprüche 1 bis 3, **dadurch gekennzeichnet, dass** man die Umsetzung bei 0 bis 60°C durchführt.

5. Verfahren nach einem oder mehreren der Ansprüche 1 bis 4, **dadurch gekennzeichnet, dass** man die Umsetzung in Anwesenheit von 20 bis 1000 Units R-Hydroxynitril Lyase/mmol Aldehyd durchführt.

6. Verfahren zur Herstellung optisch aktiver Cyanhydrine der Formel (II), 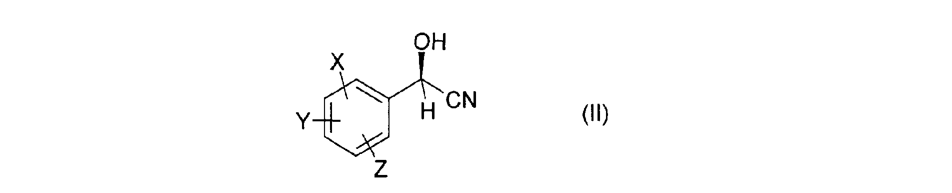 **dadurch gekennzeichnet, dass** man einen Aldehyd der Formel (I) 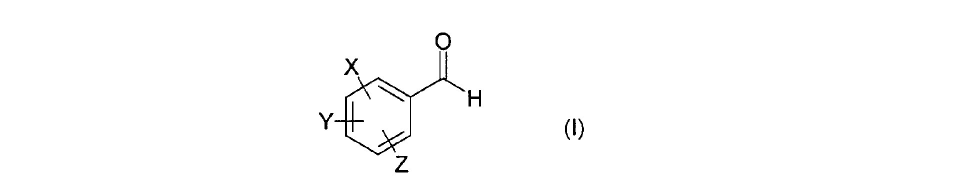 mit HCN in einem mit Wasser nicht mischbaren organischen Lösungsmittel in Anwesenheit eines wässrigen, durch Extraktion eines (R)-Hydroxynitril lyasehaltigen Naturproduktes bei pH 3,3 bis 5,5 mit Wasser, in Abwesenheit oder Anwesenheit eines Puffers, hergestellten (R)-Hydroxynitril Lyase-Extraktes unter intensiver Durchmischung bei 0 bis 60°C umsetzt, die organische Phase von der wässrigen Phase trennt und gegebenenfalls das Cyanhydrin der Formel (II) durch Hydrolyse in die entsprechende Carbonsäure überführt, wobei X, Y und Z in Formel (II) die gleiche Bedeutung wie in Formel (I) haben, unabhängig voneinander gleich oder verschieden sind und für H, F, Cl, Br, I, OH, O(C₁-C₄-Alkyl), OCOCH₃, NHCOCH₃, NO₂ oder C₁-C₄-Alkyl stehen.

7. Verfahren nach einem oder mehreren der Ansprüche 1 bis 6, **dadurch gekennzeichnet, dass** man einen Aldehyd der Formel (la) 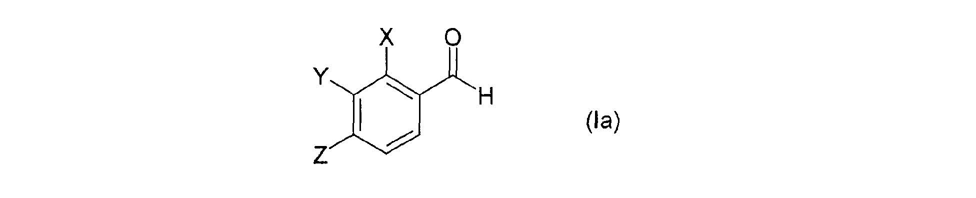 einsetzt, worin X, Y und Z die gleiche Bedeutung wie in den vorstehend genannten Formeln (I) und (II) besitzen.

8. Verfahren nach einem oder mehreren der Ansprüche 1 bis 7, **dadurch gekennzeichnet, dass** man einen Aldehyd der Formel (la), worin X für F, Cl, Br, I, OH, O(C₁-C₄-Alkyl), OCOCH₃, NHCOCH₃, NO₂ oder C₁-C₄-Alkyl und Y und Z jeweils für H stehen oder X und Y jeweils für H und Z für OH steht oder X für H und Y und Z jeweils für OH steht, einsetzt.

9. Verfahren nach einem oder mehreren der Ansprüche 1 bis 8, **dadurch gekennzeichnet, dass** man als mit Wasser nicht mischbares Lösungsmittel einen aliphatischen Ether, einen Ester einer Carbonsäure mit 1 bis 6 Kohlenstoffatomen und eines aliphatischen Alkohols mit 1 bis 4 Kohlenstoffatomen, ein aliphatisches Keton mit insgesamt 4 bis 8 Kohlenstoffatomen oder ein Gemisch derselben oder in Verdünnung mit einem aliphatischen Kohlenwasserstoff mit 4 bis 8 Kohlenstoffatomen, einem aromatischen Kohlenwasserstoff mit 7 bis 10 Kohlenstoffatomen oder einem chlorierten aliphatischen oder aromatischen Kohlenwasserstoff einsetzt.

10. Verfahren nach einem oder mehreren der Ansprüche 1 bis 9, **dadurch gekennzeichnet, dass** man als mit Wasser nicht mischbares Lösungsmittel Diethylether, Di-n-propylether, Di-i-propylether, Methyl-tert.-butylether, Di-n-butylether, Di-i-butylether oder ein Gemisch derselben einsetzt.

11. Verfahren nach einem oder mehreren der Ansprüche 1 bis 10, **dadurch gekennzeichnet, dass** man als mit Wasser nicht mischbares Lösungsmittel Diethylether, Di-n-propylether, Di-i-propylether, Methyl-tert.-butylether oder ein Gemisch derselben einsetzt.

12. Verfahren nach einem oder mehreren der Ansprüche 1 bis 12, **dadurch gekennzeichnet, dass** man je mol Aldehyd 0,8 bis 10 mol HCN einsetzt.

13. Verfahren nach einem oder mehreren der Ansprüche 1 bis 12, **dadurch gekennzeichnet, dass** man den Aldehyd in einer Konzentration von 0,1 bis 3,0 mol Aldehyd/Liter einsetzt.

14. Verfahren nach einem oder mehreren der Ansprüche 1 bis 13, **dadurch gekennzeichnet, dass** man während der gesamten Umsetzung mit HCN einen pH-Wert von 3,3 bis 5,5 einhält.

15. Verfahren nach einem oder mehreren der Ansprüche 1 bis 14, **dadurch gekennzeichnet, dass** das Gewichtsverhältnis von organischer Phase zu wässriger Phase 20:1 bis 1:20 beträgt.
